(19) Europäisches Patentamt
European Patent Office
Office européen des brevets

(11) **EP 4 438 713 A1**

(12) **EUROPEAN PATENT APPLICATION**

(43) Date of publication:
**02.10.2024   Bulletin 2024/40**

(21) Application number: **23461539.1**

(22) Date of filing: **26.03.2023**

(51) International Patent Classification (IPC):
*C12M 3/06* $^{(2006.01)}$     *C12M 1/12* $^{(2006.01)}$
*C12M 1/42* $^{(2006.01)}$     *C12M 1/00* $^{(2006.01)}$

(52) Cooperative Patent Classification (CPC):
**C12M 35/06; C12M 23/16; C12M 23/20;
C12M 25/06; C12M 25/14; C12M 25/18**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Instytut Chemii Fizycznej Polskiej
Akademii Nauk
01-224 Warszawa (PL)**

(72) Inventors:
• **Rojek, Katarzyna
01-494 Warszawa (PL)**
• **Guzowski, Jan
02-717 Warszawa (PL)**
• **Gi y ski, Konrad
05-502 Wólka Kozodawska (PL)**

(74) Representative: **Witek, Rafal
WTS Patent Attorneys
Witek, Sniezko & Partners
ul. Weigla 12
53-114 Wroclaw (PL)**

(54) **DEVICE AND METHOD FOR GENERATION OF ORDERED ARRAYS OF CELL MICROCARRIERS AT A SUBSTRATE**

(57)    The subject of the invention is a device and a system for generation of ordered arrays of cell microcarriers at a substrate.

EP 4 438 713 A1

**Description**

The subject of the invention is a device and a system for generation of ordered arrays of cell microcarriers at a substrate. The problem to be solved is to provide a device and a system capable of generating an ordered array of cell microcarriers suspended in an external hydrogel at a substrate that could be used for high-throughput dose-response studies and that could allow for the expansion of cells in the external hydrogel and for generation of a drug gradient in the hydrogel. The device and system according to the invention is easily scalable. Moreover, the problem in the state of the art is to ensure a regular spacing (distance) between the microcarriers without the use of any confining side walls.

The spatial organization of cells is critical for the physiological functioning of tissues. One of the main tasks of tissue engineering is to faithfully recreate the biological microstructure *in vitro.* The production of moldable matrices and the ability to manipulate hydrogel systems with high precision create new possibilities for spatial control of the development of cell cultures and tissues. The success of these strategies depends on faithful reproduction of the native cellular organization.

Precise modeling and design of human tissues or organs *in vitro,* e.g. in tissue regeneration and restoration therapies, requires incorporation of a network of blood vessels with a well-defined architecture. Proper vascularization of the tissue enables the supply of nutrients to all of its cells, preventing necrosis, otherwise caused by hypoxia and malnutrition. This applies in particular to constructs larger than about 1 mm [1], in which the passive diffusion of nutrients from the external cell culture medium becomes ineffective. Therefore, the formation of viable tissues requires in most cases the inclusion of a functional vascular system, a branched network of channels enabling the circulation of nutrients.

In the case of the tissues engineered for use in drug testing, and in particular cancer microtissues designed to assess the effectiveness of cytostatic agents, it is critical to design a system that would allow for the incorporation of a network of capillary vessels with controlled and reproducible morphology. This would significantly reduce the risk of obtaining inconsistent results due to differences in vascularization of model tissues. Differences in the size and geometry of the capillary network may affect the penetration of drugs inside the neoplastic tissue, and thus affect the estimated drug efficacy.

Currently, the most commonly used method of producing tissues with embedded microvasculature, used both in tissue engineering and in drug testing strategies, relies on self-organization of endothelial cells into a microscopic network of capillaries [2]. Vascular endothelial cells such as human umbilical vein endothelial cells (HUVEC) can spontaneously form tubular structures when embedded within an extracellular matrix (ECM) such as fibrin. However, the resulting vascular networks are usually random and difficult to control in terms of local morphology.

Bioprinting constitutes an important alternative enabling the controlled production of cellular structures with an embedded capillary network. The advantages are the high throughput and repeatability of the produced systems, however, the costs associated with bioprinting are high and the process itself requires specialized equipment and training, which limits its availability.

Recently, a number of devices have been proposed to generate two-dimensional arrays of cell-laden microparticles or cell aggregates of tightly controlled architecture [3-8]. Such 2D arrays constitute a highly convenient tool to study angiogenesis since they (i) allow for the precise control over the initial microparticle/cell distribution in space (ii) enable identification and tracking of the individual angiogenic 'seeds' (and the outgrowing vascular microtissues) based on their position in the array (specific X, Y coordinates), (iii) can be easily imaged using confocal microscopy. In addition, 2D arrays have been used to generate highly biomimetic, vascularized, implantable 'tissue sheets' with applications, e.g., in wound healing therapies or/and tissue restoration [4, 9, 10].

The developed methods are based either on the use of various types of geometric traps [3], or on the application of external physical fields such as acoustic [6, 7, 11, 12] or magnetic fields [5, 13-17]. Each of these methods have their advantages and limitations.

Geometric traps can be easily integrated into microfluidic channels where viscous forces guide the cells towards predesigned spots on-chip [3, 8]. However, the traps typically exploit C-shaped side walls or micro-wells, which limit the growth of vascular cellular structures within and around the trap.

Standing acoustic waves (SAWs) have been proposed as a method of generation of two-dimensional arrays of microparticles without the use of the confining walls. The method enables ordering of cells, cell clusters, colloidal particles or microdroplets above a planar substrate [6, 7, 12, 18]. However, so far, no protocol has been demonstrated that could be used directly to generate 2D arrays of eukaryotic cell-laden microparticles containing large amounts of cells (tens to hundreds) over a substrate. The current approaches focus on patterning of lines or clusters of single cells [6, 7, 18] or nanoparticles [11, 12] whereas manipulation of larger cell-laden particles (few hundred micrometers in diameter) of heterogenous structure (e.g., cell-coated surface) over a substrate remains a challenge. In fact, SAWs appear insufficient to induce movement and reorganization of such microparticles, in particular in the presence of non-vanishing friction at the substrate. One of the important limitations is also that the amplitude of SAWs cannot be increased indefinitely without affecting cell viability [18].

The attempts to use magnetic manipulation have provided new opportunities in tissue engineering, at the same time eliminating the need for use of geometric traps in a way similar to acoustic waves, yet relying on typically much simpler experimental setups. The developed approach relies on incorporation of magnetic nanoparticles into the cells/multicellular aggregates or hydrogel beads and their subsequent manipulation using external magnetic fields [5, 13-17, 19]. In the series of studies Hiroyuki Honda group [5, 13-15] introduced the so-called pin holder device allowing to generate cell culture arrays using magnetic forces at the tips of the posts protruding from a magnetized substrate. The device was used to manipulate the cells, enabling construction, e.g., of spatially controlled arrays of cell spheroids suspended in an external ECM. In particular, the authors demonstrated self-assembly of 2D arrays of endothelial cell clusters [5]. The experimental setup comprised of (i) an array of iron posts or 'pins' casted from a single slab of iron, (ii) neodymium magnet placed underneath the slab (used to magnetize the pins) and (iii) hydrophilic cell culture Petri dish with an ultrathin bottom wall (25 $\mu$m thickness). Positioning the Petri dish above the pins resulted in self-assembly of a cell-clusterarray aligned with the pins. The method had several limitations. First, generation of the iron pin holder required highly specialized and expensive equipment (e.g. a wire electrical discharge machine). Second, the system required extremely small distances between the magnetically-labeled cells and the magnetic pillars. This imposed severe restriction on the thickness of the bottom wall of the cell culture dish (the dishes with ultrathin bottom walls are commercially available, yet very expensive) and on the thickness of the hydrogel layer beneath the cells (max 50 $\mu$m), suggesting that the generated magnetic fields could be too weak to effectively induce clustering for pin-cell distances beyond around 75 $\mu$m. In particular, the forces would be insufficient in the case of the use of a standard glass coverslip as the bottom wall (thickness around 170 $\mu$m). Eventually, the method required the waiting time of approximately 30 min to allow cells to aggregate into clusters consisting of around 25 cells, and even longer times to achieve proportionally larger clusters.

An alternative approach, originally proposed by Demri et al. [17], is based on a microfabricated array of soft-magnetic NiFe (permalloy 80Ni:20Fe) micropatterns that can be magnetized by an external magnetic field and generate strong magnetic field gradients in their vicinity. The magnetic micropatterns were fabricated on a glass slide by NiFe electroplating through a photoresist mold and designed to attract magnetized cells and induce their aggregation into a cohesive spheroid. To ensure biocompatibility and avoid cell adhesion on the substrate, the magnetic patterns were spin-coated with polydimethylsiloxane (PDMS) and treated with antiadhesive solution prior to spheroid production. Despite the fact that the device was optimized towards assembly of high numbers of spheroids, it had a practical limitation in that it was not suitable for repeated use with multiple culture dishes. More specifically, the platform was constructed in such a way that the glass plate containing the magnetic hotspots (NiFe pillars) was permanently mounted to the cell-culture chamber. Also, the NiFe pillars were covered with a 50 $\mu$m thick PDMS layer such that the distance between the magnetically labelled cells and the tips of the pillars was around 50 $\mu$m. It is unclear if the method would work for thicker PDMS layers. Also, similar to the works by Honda et al. [5, 13-15], the method required rather long waiting times for cells to aggregate, which was approximately 3 hours for 35-140 cell aggregates. The resulting spheroids were highly polydisperse in size.

Last but not least, none of the thus far proposed approaches of cellular clustering into 2D arrays has been specifically designed for the purpose of generating spatially controlled microvasculatures. The approach proposed by Ino et al. [5] based on the use of a pin holder device allowed for generation of an array of small endothelial-cell (HUVEC) aggregates. However, the cells remained dispersed and freely migrated between the aggregates instead of forming the actual microvascular network. This could have been caused by the fact that the generated HUVEC clusters contained only a few cells (1 up to 7 cells per spot). Moreover, the endothelial cells were not embedded within the hydrogel, whereas such embedding is necessary to mimic the physiological conditions, in particular to recapitulate the extracellular matrix.

According to the invention, the device for generation of ordered arrays of microparticles above the substrate is characterized in that it comprises of two chips placed one above the other where the lower chip generates pre-designed magnetic field consisting of an array of magnetic 'hotspots' and the upper chip is used as a chamber accommodating the magnetic microparticles suspended in a hydrogel precursor.

The lower chip used to generate the pre-patterned magnetic field is made of polycarbonate (PC) and has the array of holes on its surface generated using CNC micromilling. The holes are designed in such a way to accommodate cylinder-shaped strong neodymium micromagnets (N≥48) magnetized along the axis. The micromagnets have to keep the height ($h$) to diameter ($d$) ratio $h/d \geq 2.5$. After the positioning, the micromagnets are sealed with a scotch tape (standard thickness, app. 30 $\mu$m -40 $\mu$m).

In the preferred example of embodiment, the said chip contains an array of $N = 5 \times 5$ cylinder neodymium N48 micromagnets of dimensions $h$ = 500 $\mu$m and $d$ =200 $\mu$m, and with the spacing between the magnets $L$ varying in the range from $L$ = 500 $\mu$m up to $L$ = 1500 $\mu$m.

Preferably, the chip used to assemble the spatially ordered arrays of microparticles is a cell culture chamber made of polydimethylsiloxane (PDMS) with the #1.5 coverslip glass thick bottom (approximately 170 $\mu$m; ISO 8255-1:2017) or thinner.

In the preferred example of embodiment, the said PDMS chip is mounted to #1.5 thick glass coverslip. To ensure efficient binding the PMDS chip and glass coverslip are first rinsed in 70% ethanol and dried under the sterile conditions.

Preferably, the two chips are positioned on top of each other with the chip containing array of the magnets placed at the bottom and mounted with gluing mass.

Preferably, the microparticles are made of polystyrene and contain ≥ 30% (w/w) of iron oxide and their diameter D obeys $1 \leq D/d \leq 1.5$.

In the preferred example of embodiment the paramagnetic polystyrene microparticles of iron oxide content of 30% and a diameter of 256 $\mu$m and neodymium magnets of 200 $\mu$m diameter were used.

The present invention discloses also a method for rapid generation of ordered arrays of microparticles over the substrate according to which the microparticles are coated with the eukaryotic cells, suspended in ECM-mimicking hydrogel, and poured into the culture chamber of PDMS chip where they assemble on top of the PC chip containing an array of micromagnets.

Preferably, the microbeads are coated with fluorescently labeled eukaryotic cells, suspended in the soft hydrogel and, after magnetic assembly, become immobilized by the crosslinking hydrogel.

Preferably, after crosslinking of the hydrogel, the PDMS chip is separated from the PC chip containing neodymium magnets and placed in the cell culture incubator. The same PC chip can be used multiple times with different PDMS chambers.

In the preferred example of embodiment, the said microbeads are coated with GFP (green fluorescent protein)-tagged HUVEC cells, suspended in fibrinogen solution, positioned over the neodymium micromagnet cylinders and immobilized after adding thrombin to the solution. After fibrinogen crosslinks, the PC chip is removed and the PDMS chip with the culture chamber containing the array of microbeads is placed in the $CO_2$ incubator. The morphology of the outgrowing microvasculature can be imaged using confocal microscopy.

Preferably, the geometry of the formed arrays of the microparticles can be regulated via adjusting the positions of the micro-milled holes in the PC chip hosting the neodymium micromagnets.

In the preferred example of embodiment, three different spacings are shown 500, 1000 and 1500 $\mu$m.

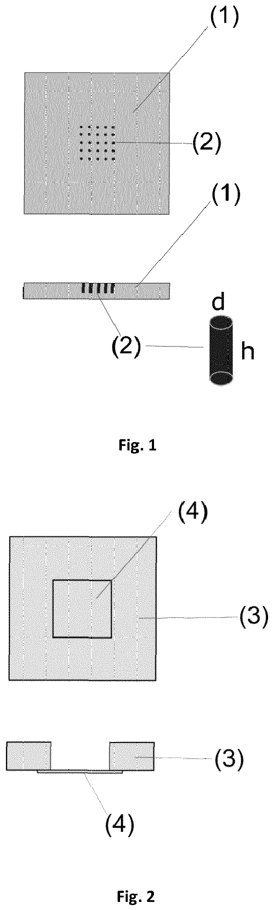

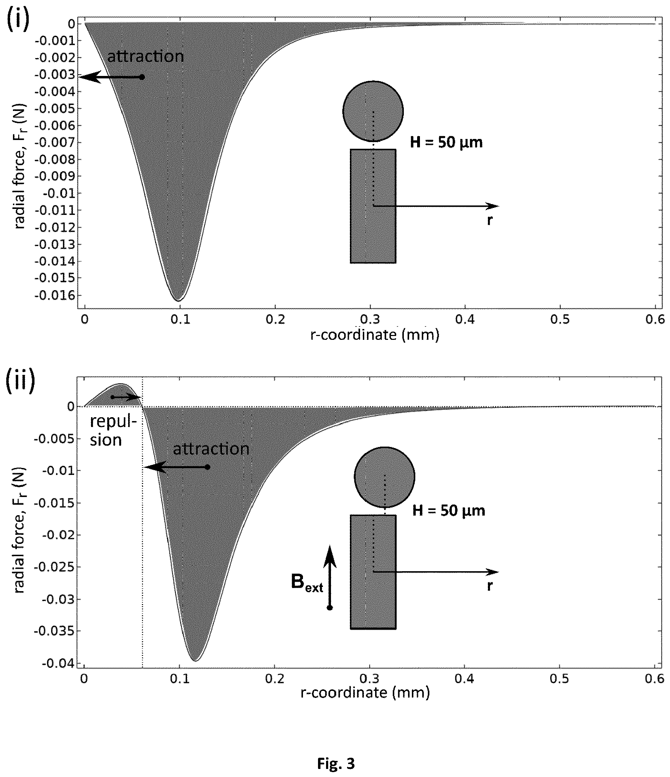

The essence of the invention is the use of strong neodymium cylinder micromagnets, which, when embedded into the PC chip (Fig. 1), serve as magnetic 'hotspots' and generate a patterned magnetic field. Due to the presence of powerful magnetic hotspots, the proposed approach allows to use relatively thick substrates, that is, for example, the conventional #1.5 coverslips of thickness of 170 $\mu$m (Fig. 2) instead for ultrathin plates or films of maximum effective thickness in the range between 25 $\mu$m and 50 $\mu$m, as in the previous studies by the Honda group [5, 13-15] or/and Demri et al. [17]. On the other hand, in the case of the use of ultrathin films, the system based on the cylindrical neodymium micromagnets also allows for more precise positioning of the microparticles as compared to the systems based on the externally magnetized iron pins of the same shape. We have performed direct numerical simulations of the magnetic field around a cylindrical micromagnet and around a cylindrical iron 'pin', the latter magnetized by a large external magnet (the situation representative of the Honda group experiments). The data (Fig. 3) show that in the case of a cylindrical micromagnet, the magnetic microparticle placed above the micromagnet is attracted to the symmetry axis of the micromagnet (the Z-axis) so that the equilibrium position of the microparticle in the XY-plane above the micromagnet is well defined. In contrast, in the case of a microparticle placed above an externally magnetized iron cylinder, when the vertical gap between the micromagnet and the particle surface is $H = 50$ $\mu$m or less, the microparticle is attracted towards a circular magnetic energy 'valley' centered around the axis of the cylinder of radius appr $r_{eq} \approx 65$ $\mu$m $\approx d/3$. In this case, the equilibrium position of the microparticle is not well defined and can be anywhere along the valley. Accordingly, in the case of the cylindrical iron pins, one could expect that the microparticle arrays assembled over the array would suffer from random perturbations in the XY-positions of the microparticles. For $H = 50$ $\mu$m, those perturbation would be of the order $r_{eq} \approx 65$ $\mu$m, resulting in relative positioning error Err$\approx \pm r_{eq}/L$, which in the case $L = 500$ $\mu$m would be around 13%. In contrast, our method based on the cylindrical micromagnets is free of this type of error.

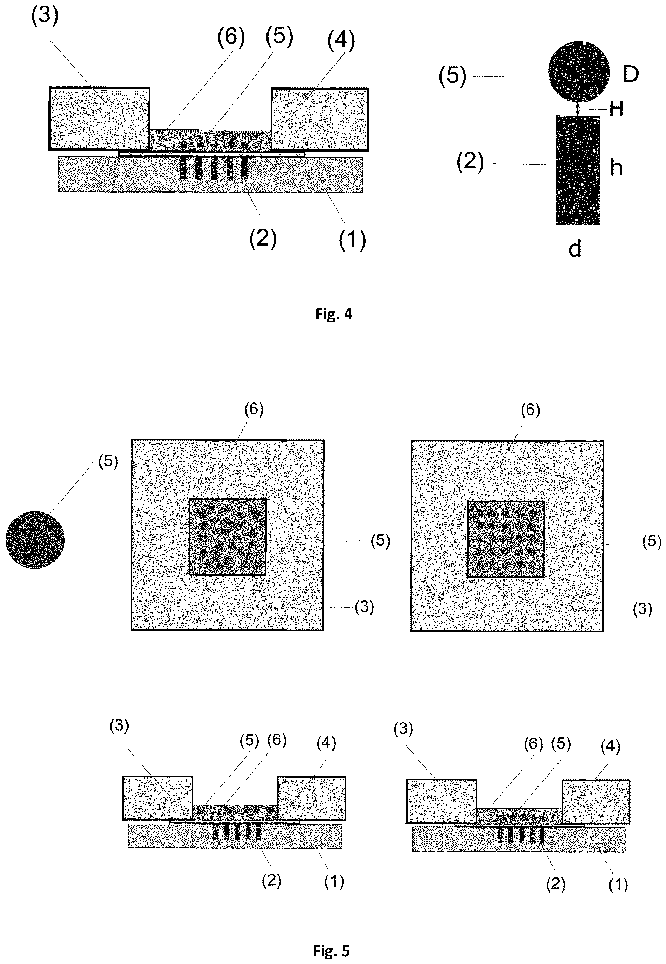

Due to the modular design of the platform, consisting of the separate reusable PC chip with the micromagnets and a disposable PDMS culture chamber chip with the usual coverslip as the bottom wall (Fig. 4), generation of arrays of microparticles can be done with high efficiency and relatively low cost without the need for expensive and highly specialized equipment (Fig. 5, Fig. 6 and Fig. 7). The arrays of microparticles are also generated within several minutes (usually 1-2 min) which is much shorter than the time required for the surrounding fibrin gel to fully crosslink (appr. 15 mins).

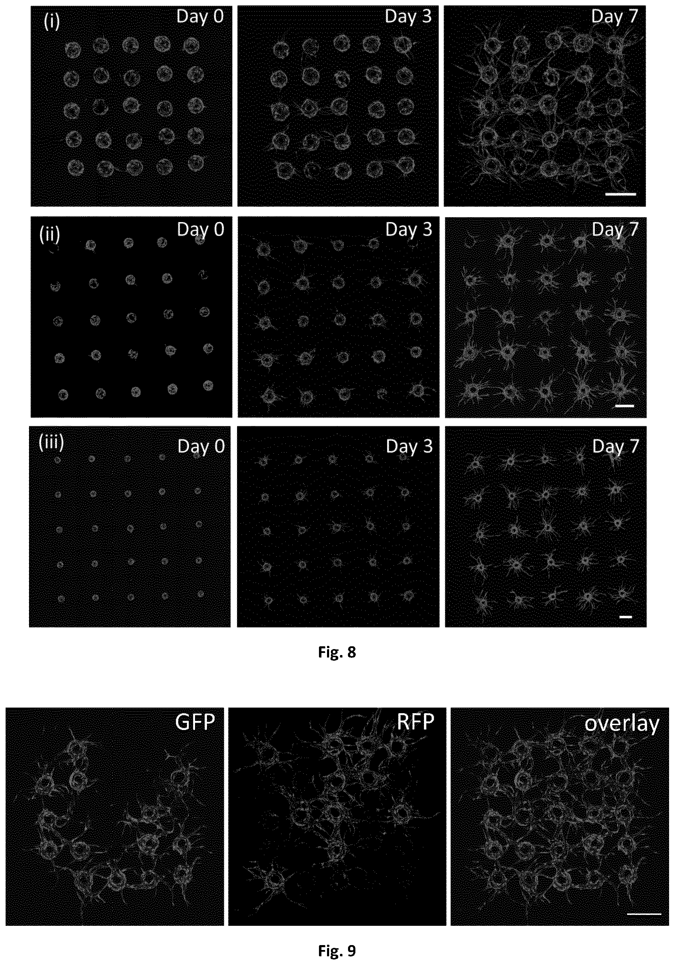

The device is unique in that it allows to assemble highly ordered arrays of microcapillary networks spanning up to 1 cm × 1 cm area or larger (Fig. 8 and Fig. 9), which can be used in the future to optimize vascularization of the tissue grafts or to develop robust screening methods of anticancer therapeutics and different anti- and pro-angiogenic agents.

The industrial applicability of the patent invention in biomedicine ranges from engineering of vascularized tissue grafts to the development of tissue-on-chip miniaturized drug screening platforms. Angiogenesis assays have received much attention in biological and pharmaceutical research in the last few decades. However, there is no available model that would allow to control and systematically investigate (i.e., in a reproducible and high-throughput manner) vascular network formation and morphology *in vitro*. Development of a device and method for production of ordered arrays of cell microcarriers above a substrate offers a range of unique applications in vascular tissue engineering. In particular, it allows assembling ordered arrays of vascular 'seeds', that is endothelial cell-coated or cell-encapsulating beads, which, under culture, give rise to arrays of sprouting microvasculatures. The invention may significantly advance the field of pharmaceutical research allowing for high-throughput screening of pro- and anti-angiogenic therapeutics in nearly identical conditions. In particular, the device allows for quantitative assessment of the impact of a drug on the morphology of each of the forming microvasculatures directly via fluorescence microscopy. Versatility of the device could be used in research involving patient-derived biological material, allowing, e.g., for (i) establishing of new treatment modalities for vascular genetic disorders, (ii) identifying of novel target genes whose expression is confined to the developing vasculature or (iii) development of personalized cancer treatments using antiangiogenic agents. The chip can remain open from above (to allow media exchange) which additionally makes it compatible with various 3D bioprinting technologies which could be used to further accelerate the assembly of the bead arrays. In the context of the development of vascularized tissues, the device may be used to optimize tissue repair therapies and lead to novel biofabrication strategies such as e.g. bioprinting with vascular seed-enriched bioinks.

Preferred examples of embodiments are now explained with reference to the accompanying figures, wherein:

Fig. 1 shows a schematic diagram of neodymium-micromagnet cylinder array embedded in PC chip.

Fig. 2 presents schematic diagram illustrating the PDMS chamber with the glass coverslip bottom.

Fig. 3 presents the results of numerical simulation (using COMSOL Multiphysics software) of the horizontal component of the magnetic force experienced by a magnetic microparticles placed at the height $H$ = 50 $\mu$m (i) above a micromagnet, and (ii) above an iron cylinder magnetized by an external large cylindrical neodymium magnet of height 5 mm, diameter 20 mm, and placed underneath the iron cylinder at the surface-to-surface distance of 1.5 mm.

Fig. 4 presents a schematic diagram illustrating the arrangements of the two chips.

Fig. 5 shows the assembly of an ordered array of microparticles coated with eukaryotic cells.

Fig. 6 shows images illustrating two-step assembly of an ordered array of microparticles involving magnetic self-assembly followed by manual corrections performed using titanium fine tip tweezers.

Fig. 7 presents experimental images illustrating different spacing of beads in 2D arrays.

Fig. 8 shows experimental images illustrating development of capillary network between the GFP-tagged endothelial cell-coated microbeads seeded at different spacing, scale bar 500 $\mu$m.

Fig. 9 shows images illustrating development of capillary network between the GFP and RFP-tagged endothelial cell-coated microbeads, scale bar 500 $\mu$m.

Fig. 10 presents a schematic diagram illustrating the device designed to generate of 24 separate arrays of microparticles, consistent with 24-well plate format.

Fig. 11 presents a scheme of the device with the concentration-gradient generator.

**Example 1**

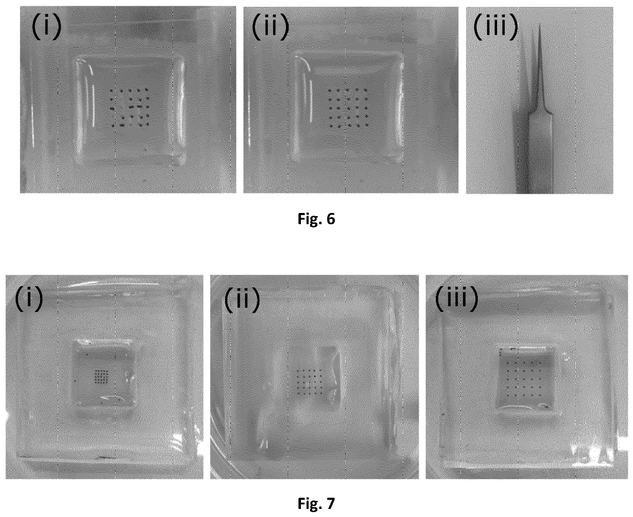

The device described here is composed of 25 mm × 25 mm PC (PolyCarbonate) chip (1), which is the upper layer, with embedded $M = N \times N = 5 \times 5$ array of cylinder-shaped neodymium micromagnets grade N48, $d$=200 $\mu$m, $h$=500 $\mu$m (2) (Fig. 1) at spacings $L$, and a 30 mm × 30 mm PDMS chip (3), which is the bottom layer, with a culture chamber (6) of the dimensions 10 mm × 10 mm and with the 24 mm × 24 mm 170 $\mu$m thick #1.5 glass coverslip (4) bottom (Fig. 2). Before bonding, the glass coverslip and the PDMS chip are rinsed in 70% ethanol and dried under the biological safety cabinet. The global shape of the array of micromagnets is not limited to the one above, which serves only as an example, however the spacing between micromagnets should be regular, preferably periodic. The skilled person will be able to provide any global shape depending on the need. However, in general, the arrays of very high or low aspect ratios are discouraged due to the expected lower efficiency of the magnetic assembly of the microparticle arrays. The global shape of the culture chamber (6) is also not limited to the abovementioned square shape but should be design in such a way that the distance between outer beads in the array and the wall of the chamber spans at least 1000 $\mu$m allowing for undisturbed growth of the EC sprouts. To accommodate an array of neodymium cylinder-shaped micromagnets in PC chip, first, the array of holes is fabricated on its surface using CNC micromilling according to the chosen design. Subsequently, the PC chip is placed on top of a large neodymium magnet, i.e., preferably of diameter $d'$ exceeding the size of the array ($d' > NL$) and of thickness $> d'/2$. The chip is observed under a stereoscopic microscope and micromagnets are manually positioned inside the holes using non-magnetic titanium fine tip tweezers. The spacing between the micromilled holes determines the spacing between the neodymium micromagnets which in turn governs the architecture of the outgrowing capillary network. The device is assembled in such a way that the PDMS chip is mounted on top of the PC chip (Fig. 4), tightly aligned with the gluing paste, e.g. UHU patafix to avoid any displacements, and placed under the stereoscopic microscope. The distance between the paramagnetic polystyrene microparticles containing 30% of iron oxide (5) and the cylinder-shape neodymium micromagnets (2) $H$ should be equal or smaller than 170 $\mu$m which is the thickness of #1.5 (ISO norm 8255-1:2017) glass coverslip. Prior to the assembly of the arrays the paramagnetic polystyrene microparticles of 256 $\mu$m diameter (5) are coated with GFP-tagged HUVECs (Fig 4). To this end, microparticles are suspended in cell culture medium and incubated with GFP-HUVEC cells for 4 h with gentle agitation, and subsequently left overnight in the incubator to recover. The next day, GFP-HUVEC coated microbeads are transferred to another Eppendorf tube and resuspended in 2.5 mg/mL fibrinogen solution mixed human dermal fibroblasts (hDFs). The role of hDFs is to enhance the angiogenic behavior of HUVECs. Here, 25 microbeads are resuspended in 180 μl of 2.5 mg/mL fibrinogen solution, mixed with 100 000 hDFs and poured in the culture chamber (6) of the PDMS chip (Fig 5). After positioning of the PDMS chip containing the polystyrene microparticles above the PC chip containing the array of cylinder-shape neodymium micromagnets, fibrinogen solution is aspirated to an Eppendorf tube, mixed with 20 μl of 6.25U/mL thrombin solution and poured back into the culture chamber of the PDMS chip allowing the hydrogel to crosslink (1-2 minutes). The array generation process consists of two stages. Immediately after pouring of the hydrogel solution containing the microparticles into the PDMS culture chamber (6), approximately 80% of microparticles self-assemble over the magnetic hotspots. The remaining microparticles are manually positioned using non-magnetic titanium fine tip tweezers (Fig. 6). Due to the necessity of manual corrections and the relatively short time of hydrogel crosslinking, the maximum size of a perfectly aligned array is in practice limited to around $M_{max}$ = 100 microparticles as explained below.

Preferably, the array of micromagnets is a square array and the chamber (6) is also square, however, arbitrary array designs are possible as long as the distance between the neighboring micromagnets $L$ obeys 1500 μm > $L$ > 1.5d, where $d$ is the diameter of the micromagnets. The lower bound is a consequence of the tendency of the beads to aggregate under the external magnetic forces if the distance between the micromagnets is too small, while the upper bound is associated with the typical maximal reach of the capillary networks outgrowing from each of the beads. In general, the maximal size of the array $M_{max}$ is limited by the number $n$ of initially misplaced beads that occasionally fail to self-assemble into an ordered array under magnetic forces. These beads need to be manually re-positioned within the time of hydrogel crosslinking. Here, the soluble fibrinogen is converted enzymatically by thrombin to fibrin-based clot. In the case of the 2.5 mg/mL fibrin gel the crosslinking time at room temperature is around 2-3 mins which allows to perform around $n$ = 20 manual corrections. In practice, the fraction of the misplaced beads oscillates around 20% so that the maximal size of the array $M_{max}$ equals roughly $M_{max} \approx n/0.2 = 20/0.2 = 100$. In principle, $M_{max}$ could be increased, e.g., via the use of a hydrogel precursor with a longer crosslinking time.

Figure 7 presents the assembled arrays with different spacings of the microbeads (i) 500 μm, (ii) 1000 μm, (iii) 1500 μm. Subsequently, the PDMS chip is separated from PC chip, placed inside the cell culture petri dish and put in the $CO_2$ incubator (37°C, 5% $CO_2$). After 15 minutes when the fibrin is fully crosslinked, the 0.7 mL of fresh cell culture medium is added on top of the hydrogel. Generated arrays can be cultured for approximately 2 weeks (Fig. 8). Figure 8 shows confocal images, acquired at different times-points, showing GFP-HUVEC-coated microbeads seeded at three different spacings: (i) 500 μm, (ii) 1000 μm and (iii) 1500 μm, illustrating the development of the microvasculatures. Figure 9 demonstrates the possibility of generation of double-species microvasculatures in which some of the beads are coated with a differently labeled of HUVEC cells. Here, we show an array consisting of subpopulations of GFP and RFP -tagged HUVEC-coated microbeads.

## Example 2

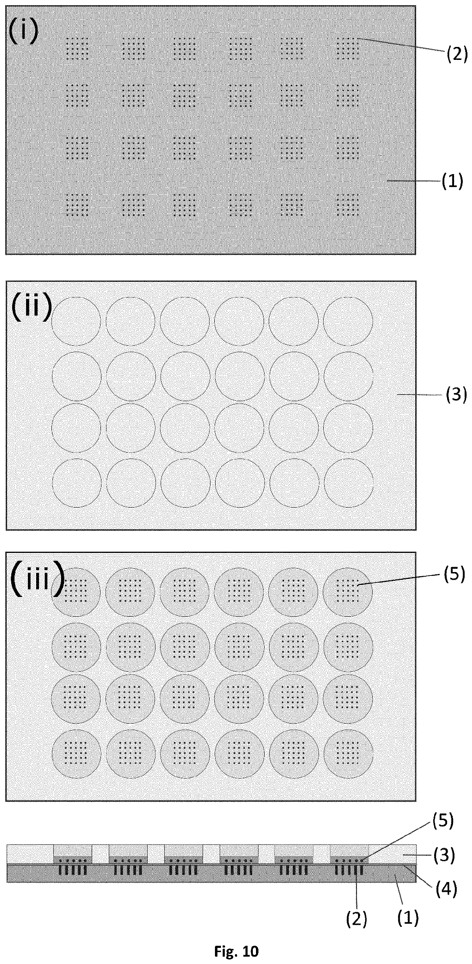

We also present an integrated system consisting of an array of neodymium micromagnet sub-arrays that could be used for generation of multiple microparticle sub-arrays and as such increase the throughput of the assay (Fig. 10). The device comprises (i) a 127 mm × 85 mm PC chip (1) (corresponding to the outer dimensions of a typical multi-well plate) with an embedded 6 × 4 array of neodymium micromagnet sub-arrays (2), each sub-array containing 5 × 5 = 25 micromagnets, and (ii) a multi-well plate, here the 24-well plate (3), with the coverslip thick bottom (4). The neodymium micromagnet arrays embedded in the PC chip are positioned each underneath a well. Depending on the intended use of the system, it is possible to adjust the system to various multi-well plate formats. Figure 9 illustrates a 24-well plate used to simultaneously generate 24 arrays of microparticles, altogether capable of manipulating 6 × 4 × 5 × 5 = 600 microparticles. In particular, standardized multi-well plate formats allow for convenient imaging and its automation.

## Example 3

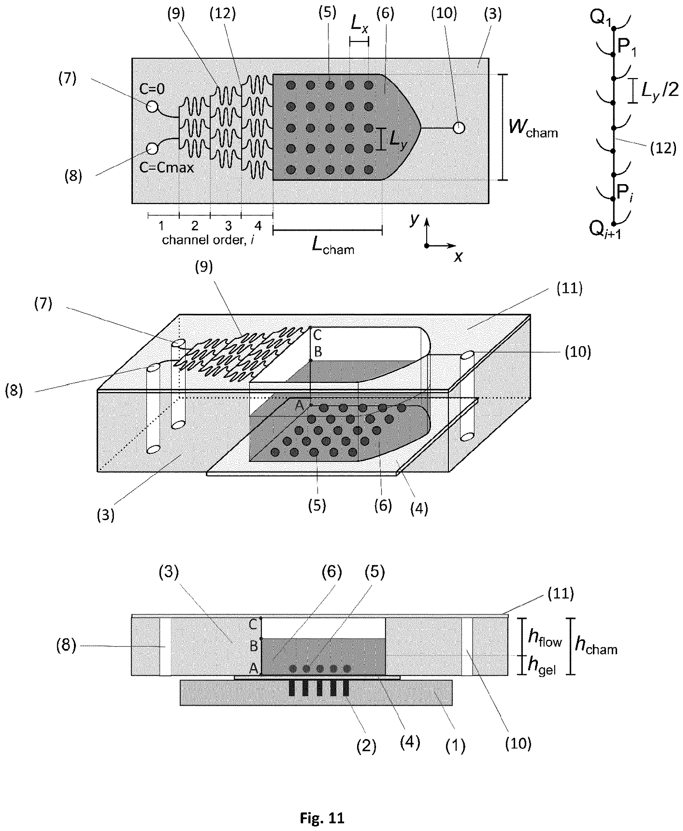

Below we present an integrated system capable of generating a drug gradient over the microparticle array that could be used for high-throughput dose-response studies. Figure 11 presents a scheme of a concentration gradient-generating PDMS microdevice composed of (i) two inlets (7 and 8), one delivering low- or zero drug concentration ($C$ = $C_{min}$) and the other maximum concentration ($C$ = $C_{max}$), (ii) a forked network of microfluidic microchannels (9) used to generate a concentration gradient at the outlets [20], (iii) a cell-culture chamber (6) and (iv) an outlet for waste removal (10). The microchannels (9) in the network have all the same width $w_{mc}$ = 120 μm and height $h_{mc}$ = 120 μm. The network consists of $n_1$ = 2 first-order channels (supplied from the two independent inlets 7, 8) forked into $n_2 = n_1 + 1 = 2 + 1 = 3$ second-order channels forked into $n_3 = n_2 + 1 = 3 + 1 = 4$ third-order channels, and so on, such that the ($i$-1)-th forking

produces $n_i = i + 1$ outlet channels, where $i = 2,...,N_y$-1 and the directions $x$ (longitudinal) and $y$ (lateral) are here defined as the parallel and the perpendicular, respectively, to the flow direction in the chamber. The $(i$-1)-th forking is constructed such that the $(i$-1)-th order microchannels connect to a single perpendicular channel (12) of length $L_{perp,i} = iL_y$ at points $P_1,...,P_i$ which are equidistant at spacing $L_y$ (equal to the expected lateral spacing between microparticles in the chamber) and the $i$-th order microchannels connect to the same perpendicular channel at points $Q_1,...,Q_{i+1}$ which are also equidistant at spacing $L_y$ such that the points $Q_1$ and $Q_i$ are the terminal points of the perpendicular channel. The spacing between the neighboring points $P_j$ and $Q_j$ is always constant and equal $L_y/2$ where $j = 1,...,i$. The number of outlet channels at the end of the network matches the lateral size of the microparticle array $N_y$.

The generation of the gradient is based on the diffusion of the drug in the direction lateral to the flow across the microchannels within the network. Therefore, in order to allow generation of the gradient, the length $L_{mc}$ of the microchannels between bifurcations must be large enough to allow the diffusion of typical water-soluble species across the microchannel width under the applied rates of flow $q_1$ and $q_2$ supplying the drug solution ($C_{max}$) and the pure buffer (or medium, $C_{min}$), respectively. Preferably, $q_1 = q_2 = q$ so that the net rate of flow is $q_{tot} = q_1 + q_2 = 2q$. The condition for diffusion can be expressed as $t_{diff} < t_{flow}$, where $t_{diff} = w_{mc}^2/D_0$ is the time of diffusion across the microchannel, with $D_0$ being the diffusion coefficient, and where $t_{flow} = L_{mc}/(2q/(i+1)/w_{mc}^2)$ is the time of flow along the $i$-th order microchannel. The length $L_{mc}$ of the $i$-th order microchannel is therefore limited by the inequality:

$$L_{mc} > 2q/D_0/(i+1).$$

The strongest constraint on $L_{mc}$ corresponds to $i = 1$. Since in the device the lengths $L_{mc}$ are independent of $i$, the minimal length of the microchannel can be expressed as:

$$L_{mc,min} = q/D_0.$$

We take $q = 10$ nL/s which corresponds to reasonably small consumption of reagents (mostly the drug) even during the long-term culture. In particular, the consumption equals 864 μL of the supplied drug solution after 1 day of perfusion; accordingly, it is less than 5 mL after 5 days of perfusion. Taking the typical value $D_0 = 10^{-5}$ cm$^2$/s = $10^{-3}$ mm$^2$/s we finally get,

$$L_{mc,min} = 1 \text{ cm}$$

Therefore, preferably $L_{mc} = 1$ cm for microchannels of all orders.

The network of microchannels produces at the outlet a series of $N_y$ streams of a drug solution with a linearly decreasing concentration of the drug, starting from $C = C_{min}$ up to $C = C_{max}$. In particular,

$$C_j = C_{min} + (C_{max} - C_{min})(j-1)/(N_y-1), \qquad (*)$$

where $j = 1,...,N_y$ labels the outlets starting from the side at which $C = C_{min}$ is applied.

To generate an array of microparticles within the cell culture chamber, the PDMS chip (3) is placed above the PC chip with an embedded neodymium micromagnet array. Fibrin solution with microparticles is poured into PDMS chamber of depth $h_{cham} = 1.5$ mm allowing a hydrogel layer of a thickness of approximately $h_{gel} = 1.0$ mm (the distance between points A and B) to form, leaving a space of approximately $h_{flow} = h_{cham} - h_{gel} = 0.5$ mm height (the distance between points B and C) to supply of the drug-containing culture medium. The lateral distance between the micromagnets $L_y$ in the PC chip, and so the lateral distance between the microparticles in the PDMS chamber, should be set such that $L_y$ is significantly larger than $h_{cham}$, preferably $L_y = 3.5$ mm. This condition warrants that the HUVEC cells coating the microparticles in row $j$ experience the same (or nearly the same) drug concentration as the concentration $C_j$ generated at the outlet $j$ of the microchannel network (eq. *).

Before media perfusion, the PDMS chip is closed from the top with another glass coverslip (11). Bonding is facilitated by exposing the PDMS chip to oxygen plasma shortly before the experiment. After bonding, the flow of the two drug-containing medium solutions ($C = C_{min}$ and $C = C_{max}$) is supplied to the two inlets.

The cell-culture chamber is constructed such to accommodate the array of $N_x \times N_y$ microparticles evenly distributed at spacings $L_x$ and $L_y$ in the longitudinal and lateral directions, respectively, where preferably $N_y = 5$. The width of the chamber equals $W_{cham} = N_yL_y$. The distance between the peripheral outlet channels of the microchannel network and the lateral chamber walls must be the same at both sides of the chamber and equal to $L_y/2$. The chamber terminates with a narrowing connected with the outlet (10).

The length of the part of the chamber containing the microparticles, $L_{cham}$, must be short enough *not* to allow for the diffusion of water soluble species at the lateral distance $L_y$ separating the microparticles upon perfusion of the chamber. Otherwise the HUVEC cells coating the microparticles would experience different drug concentrations than those generated at the outlets of the microchannel network. The requirement of limited diffusion in this case can be written as $t_{diff} > t_{flow}$, where $t_{diff} = L_y^2/D_0$ is the time of diffusion across one row of particles and $t_{flow} = L_{cham}/(2q/(N_yL_y)/h_{flow})$ is the time of flow along the chamber. This leads to:

$$L_{cham} < 2L_y q/(N_y h_{flow} D_0).$$

Taking $L_y$ = 3.5 mm, $h_{flow}$ = 0.5 mm and $N_y$ = 5 we obtain $L_{cham} < L_{cham,max}$ with

$$L_{cham,max} = 2.8 \times q/D_0.$$

Taking the same $q$ and $D_0$ as above, we get

$$L_{cham,max} = 2.8 \text{ cm}.$$

Preferably, $L_{cham}$ = 1.5 cm. Preferably, we also have $W_{cham} = N_y L_y$ = 5 × 3.5 mm = 1.75 cm. Accordingly, in such a case, the chamber has an almost square shape, which is advantageous because of the ease of magnetic assembly of the microparticle array. The longitudinal size of the array $N_x$ consistent with the abovementioned chamber dimensions, assuming that $L_x$ = 15 mm, equals $N_x = L_{cham}/L_x$ = (15 mm)/(1.5 mm) = 10. In terms of drug screening applications, the number of biological repetitions N = 10 can be considered statistically relevant. It could be further increased up to the maximal value $N_{x,max}$ = floor($L_{cham,max}/L_x$) = floor((28 mm)/(1.5 mm)) = 18. This would come at a cost of almost twice longer chamber array which could slightly hamper the efficiency of the magnetic assembly of the microparticle array.

The drug is supplied over the hydrogel layer at a translational velocity $U$ which can be calculated as $U = 2q/N_y/h_{flow}/L_y$. For the preferred dimensions of the array and the chamber mentioned above and for $q$ = 10 nL/s we obtain $U$ = 2.3 $\mu$m/s, which is advantageous because it is within the range of physiological interstitial flows.

Overall, the integration of the gradient-generating network with the culture chamber allows for fast, straightforward and cost-effective dose-response studies, e.g., drug screening tests, using small amounts of reagents.

The approach is of great value in terms of screening of the anti-angiogenic agents in cancer treatment (until today the US Food and Drug Administration has approved the total of 14 such drugs) or pro-angiogenic therapeutics that could support wound healing and tissue restoration therapies. The use of an ordered array of 'angiogenic seeds' warrants homogenous culture conditions (including spatial restrictions) whereas, at sufficiently large seed-seed spacing (in practice 1.5 mm or larger), each seed can be treated as an independent experiment. In particular, as the gradient-generating device produces $N_y$ drug concentrations, the array of microparticles of dimensions $N_x \times N_y$ should be used for drug testing. I such a case one obtains $N_x$ biological repetitions of the condition corresponding to each of the $N_y$ generated drug concentrations which equals to the number of outlets of the bifurcated microchannel network. The effect of a drug can be monitored in terms of the resulting morphology of the vasculature quantified in terms of several metrics [21-23] such as, e.g., (i) the area and length of the developed vascular network, (ii) the number of branches including the primary branches, (iii) the number of tips or (iv) the average width of the branches.

**References:**

1. Shirure, V.S., C.C.W. Hughes, and S.C. George, Engineering Vascularized Organoid-on-a-Chip Models. Annu Rev Biomed Eng, 2021. 23: p. 141-167.

2. Tronolone, J.J. and A. Jain, Engineering new microvascular networks on-chip: ingredients, assembly, and best practices. Adv Funct Mater, 2021. 31(14): p. 2007199.

3. Manzoor, A.A., L. Romita, and D.K. Hwang, A review on microwell and microfluidic geometric array fabrication techniques and its potential applications in cellular studies. Canadian Journal of Chemical Engineering, 2021. 99(1): p. 61-96.

4. Song, W., et al., Engineering transferrable microvascular meshes for subcutaneous islet transplantation. Nature Communications, 2019. 10: p. 4602.

5. Ino, K., M. Okochi, and H. Honda, Application of magnetic force-based cell patterning for controlling cell-cell interactions in angiogenesis. Biotechnol Bioeng, 2009. 102(3): p. 882-90.

6. Armstrong, J.P.K., et al., Engineering Anisotropic Muscle Tissue using Acoustic Cell Patterning. Adv Mater, 2018. 30(43): p. e1802649.

7. Armstrong, J.P.K., et al., Spatiotemporal quantification of acoustic cell patterning using Voronoi tessellation. Lab Chip, 2019. 19(4): p. 562-573.

8. Di Carlo, D., L.Y. Wu, and L.P. Lee, Dynamic single cell culture array. Lab Chip, 2006. 6(11): p. 1445-9.

9. Hakimi, N., et al., Handheld skin printer: in situ formation of planar biomaterials and tissues. Lab on a Chip, 2018. 18(10): p. 1440-1451.

10. Kobayashi, A., et al., Preparation of stripe-patterned heterogeneous hydrogel sheets using microfluidic devices for high-density coculture of hepatocytes and fibroblasts. Journal of Bioscience and Bioengineering, 2013. 116(6): p. 761-767.

11. Tian, L., et al., Spontaneous assembly of chemically encoded two-dimensional coacervate droplet arrays by acoustic wave patterning. Nat Commun, 2016. 7: p. 13068.

12. Nichols, M.K., et al., Fabrication of Micropatterned Dipeptide Hydrogels by Acoustic Trapping of Stimulus-Responsive Coacervate Droplets. Small, 2018. 14(26): p. e1800739.

13. Okochi, M., et al., Three-dimensional cell culture array using magnetic force-based cell patterning for analysis of invasive capacity of BALB/3T3/v-src. Lab Chip, 2009. 9(23): p. 3378-84.

14. Okochi, M., T. Matsumura, and H. Honda, Magnetic force-based cell patterning for evaluation of the effect of stromal fibroblasts on invasive capacity in 3D cultures. Biosens Bioelectron, 2013. 42: p. 300-7.

15. Yamamoto, S., et al., Effect of vascular formed endothelial cell network on the invasive capacity of melanoma using the in vitro 3D co-culture patterning model. PLoS One, 2014. 9(7): p. e103502.

16. Lin, R.Z., et al., Magnetic reconstruction of three-dimensional tissues from multicellular spheroids. Tissue Eng Part C Methods, 2008. 14(3): p. 197-205.

17. Demri, N., et al., Remote Magnetic Microengineering and Alignment of Spheroids into 3D Cellular Fibers. Advanced Functional Materials, 2022. 32(50): p. 2204850

18. Levario-Diaz, V., et al., Effect of acoustic standing waves on cellular viability and metabolic activity. Sci Rep, 2020. 10(1): p. 8493.

19. Neto, A.I., et al., Fabrication of Hydrogel Particles of Defined Shapes Using Superhydrophobic-Hydrophilic Micropatterns. Adv Mater, 2016. 28(35): p. 7613-9.

20. Jeon, N.L., et al., Generation of solution and surface gradients using microfluidic systems. Langmuir, 2000. 16(22): p. 8311-8316.

21. Bezenah, J.R., Y.P. Kong, and A.J. Putnam, Evaluating the potential of endothelial cells derived from human induced pluripotent stem cells to form microvascular networks in 3D cultures. Scientific Reports, 2018. 8: p. 2671.

22. Ghajar, C.M., et al., The effect of matrix density on the regulation of 3-D capillary morphogenesis. Biophysical Journal, 2008. 94(5): p. 1930-1941.

23. Newman, A.C., et al., The requirement for fibroblasts in angiogenesis: fibroblast-derived matrix proteins are essential for endothelial cell lumen formation. Molecular Biology of the Cell, 2011. 22(20): p. 3791-3800.

## Claims

1. Device for generating an ordered array of cell microcarriers, **characterized in that**, it comprises a bottom layer (1) and an upper layer (3), wherein each of the layers (1, 3) has an upper surface and a bottom surface, and between the bottom surface of the upper layer (3) and the upper surface of the bottom layer (1) there is a spacer (4) of thickness H, and the bottom layer (1) from the side of the upper surface comprises a system of M micromagnets (2) with a diameter of d $\mu$m and a height of h $\mu$m, wherein the ratio h/d $\geq$ 2.5, and the upper layer (3) comprises at least one opening constituting the culture chamber (6), wherein the shape of the culture chamber (6) corresponds to the shape of the system of micromagnets (2), wherein the distance between the micromagnets is from 1.5d to 1500$\mu$m, and the culture chamber (6) comprises a layer of hydrogel comprising polymeric superparamagnetic beads (5) with a diameter of D $\mu$m, where the beads (5) are placed above the micromagnets (2) at a distance equal H, wherein one bead (5) corresponds to each micromagnet (2).

2. Device according to claim 1, **characterized in that**, the bottom layer (1) is made of polycarbonate.

3. Device according to claim 1, **characterized in that**, the upper layer (3) is made of PDMS.

4. Device according to claim 1, **characterized in that**, the polymeric paramagnetic beads (5) are made of polystyrene and comprise not less than 30% (w/w) of iron oxide, wherein the diameter of the beads D is determined by the expression $1 \leq D/d \leq 1.5$.

5. Device according to claim 1, **characterized in that**, the diameter d of the micromagnet in the system of micromagnets (2) is d = 200 $\mu$m.

6. Device according to claim 1, **characterized in that**, the hydrogel layer is obtained by cross-linking of fibrin deposited in the culture chamber.

7. Device according to claim 1, **characterized in that**, the bottom layer (1) and the spacer (4) are connected by an adhesive while the upper layer (3) and the spacer (4) are irreversibly bonded to provide a leak-proof connection.

8. Device according to any of claims 1, 2, 5 **characterized in that**, the micromagnets (2) are placed in the bottom layer (1) in holes made by micro-drilling.

9. Device according to claims 1 or 4, **characterized in that**, the beads (5) are covered with a layer of green fluorescent protein tagged human umbilical vein endothelial cells.

10. Generating system for forming a microcarrier array, **characterized in that**, it comprises at least one device for forming a microcarrier array as defined in the claim 1.

11. Microfluidic device for high-throughput dose-response testing under the gradient of an active substance, **characterized in that**, it comprises a device for forming a microcarrier array as defined in claim 1, wherein the upper layer (3) comprises first liquid inlet (7) for introducing the test solution without active substance and the second liquid inlet (8) for introducing the test solution with the active substance connected to a hierarchically forked system of micro-channels (9) with outlets in the culture chamber (6), and the culture chamber (6) is connected to the outlet (10) through a microchannel, and on the upper layer (1) facing the upper surface the device comprises a closure layer (11).

12. Microfluidic device according to claim 11, **characterized in that**, the forked microchannel system (9) comprises $N_y$ outlets in the culture chamber (6).

13. Microfluidic device according to any of claims 11-12, **characterized in that**, the number $N_y$ of microchannel outlets (9) in the culture chamber (6) is determined by the expression $W_{cham} = N_y L_y$, where $L_y$ is the distance between the micromagnets (2) in the culture chamber (6) in the lateral direction and $W_{cham}$ is the width of the culture chamber (6).

14. Microfluidic device according to any of claims 11-13, **characterized in that**, each row of channels in a hierarchically forked system of micro-channels (9) has one longitudinal channel more than the preceding row, wherein the first row comprises two longitudinal channels, wherein each longitudinal channel in the first row is connected to one liquid inlet (7, 8) and the rows of longitudinal channels are connected by a perpendicular channel (12), which is the outlet of the longitudinal channels from the previous row and the inlet of the longitudinal channels from the next row.

15. Microfluidic device according to any of claims 11-14, **characterized in that**, the length of the microchannel (9) in a single row is determined according to the expression $L_{mc} > 2q/Do/(i+1)$, where $L_{mc}$ is the length of the microchannel, q is the volumetric flow rate, $D_0$ is the diffusion coefficient, i is the microchannel order, wherein preferably the length of the culture chamber (6) is determined according to the expression $L_{cham} < 2L_y q/(N_y h_{flow} D_0)$, where $L_{cham}$ is the length of the culture chamber, q is the volumetric flow rate, $N_y$ is the number of microchannel outlets (9), $L_y$ is lateral distance separating microparticles, $D_0$ is the diffusion coefficient, $h_{flow} = h_{cham} - h_{gel}$, where $h_{cham}$ is the height of the chamber and $h_{gel}$ is the thickness of the hydrogel layer, and where preferably $h_{cham} = 1.5$ mm and $h_{gel} = 1$ mm.

(1)

(2)

(1)

d

(2) h

**Fig. 1**

(4)

(3)

(3)

(4)

**Fig. 2**

**Fig. 3**

**Fig. 4**

**Fig. 5**

Fig. 6

Fig. 7

Fig. 8

Fig. 9

Fig. 10

**Fig. 11**

Europäisches
Patentamt

European
Patent Office

Office européen
des brevets

# EUROPEAN SEARCH REPORT

Application Number

**EP 23 46 1539**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MINA OKOCHI ET AL: "Three-dimensional cell culture array using magnetic force-based cell patterning for analysis of invasive capacity of BALB/3T3/v-src", LAB ON A CHIP, vol. 9, no. 23, 1 January 2009 (2009-01-01), page 3378, XP055405661, UK ISSN: 1473-0197, DOI: 10.1039/b909304d * the whole document * | 1-15 | INV. C12M3/06 C12M1/12 C12M1/42 C12M1/00 |
| A | YAMAN SENA ET AL: "Magnetic Force-Based Microfluidic Techniques for Cellular and Tissue Bioengineering", FRONTIERS IN BIOENGINEERING AND BIOTECHNOLOGY, vol. 6, no. 192, 19 December 2018 (2018-12-19), pages 1-29, XP055875859, DOI: 10.3389/fbioe.2018.00192 * abstract * * pages 1-3 * * pages 10, 11; figure 3 * | 1-15 | |
| A | CN 109 609 344 A (NINGBO MEIJING MEDICAL TECH CO LTD) 12 April 2019 (2019-04-12) * the whole document * | 1-15 | TECHNICAL FIELDS SEARCHED (IPC) C12M |

-/--

The present search report has been drawn up for all claims

| Place of search | Date of completion of the search | Examiner |
|---|---|---|
| The Hague | 13 September 2023 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

**EP 23 46 1539**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ABEDINI-NASSAB ROOZBEH ET AL: "A Microfluidic Platform Equipped With Magnetic Nano Films for Organizing Bio-Particle Arrays and Long-Term Studies", IEEE SENSORS JOURNAL, IEEE, USA, vol. 20, no. 17, 4 May 2020 (2020-05-04), pages 9668-9676, XP011803178, ISSN: 1530-437X, DOI: 10.1109/JSEN.2020.2992551 [retrieved on 2020-08-04] * abstract * * page 9668 – page 9669; figure 1 * ----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 13 September 2023 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 23 46 1539

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2023

| Patent document cited in search report | | Publication date | Patent family member(s) | Publication date |
|---|---|---|---|---|
| CN 109609344 | A | 12-04-2019 | NONE | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHIRURE, V.S. ; C.C.W. HUGHES ; S.C. GEORGE.** Engineering Vascularized Organoid-on-a-Chip Models. *Annu Rev Biomed Eng,* 2021, vol. 23, 141-167
- **TRONOLONE, J.J. ; A. JAIN.** Engineering new microvascular networks on-chip: ingredients, assembly, and best practices. *Adv Funct Mater,* 2021, vol. 31 (14), 2007199
- **MANZOOR, A.A. ; L. ROMITA ; D.K. HWANG.** A review on microwell and microfluidic geometric array fabrication techniques and its potential applications in cellular studies. *Canadian Journal of Chemical Engineering,* 2021, vol. 99 (1), 61-96
- **SONG, W. et al.** Engineering transferrable microvascular meshes for subcutaneous islet transplantation. *Nature Communications,* 2019, vol. 10, 4602
- **INO, K. ; M. OKOCHI ; H. HONDA.** Application of magnetic force-based cell patterning for controlling cell-cell interactions in angiogenesis. *Biotechnol Bioeng,* 2009, vol. 102 (3), 882-90
- **ARMSTRONG, J.P.K. et al.** Engineering Anisotropic Muscle Tissue using Acoustic Cell Patterning. *Adv Mater,* 2018, vol. 30 (43), e1802649
- **ARMSTRONG, J.P.K. et al.** Spatiotemporal quantification of acoustic cell patterning using Voronoi tessellation. *Lab Chip,* 2019, vol. 19 (4), 562-573
- **DI CARLO, D. ; L.Y. WU ; L.P. LEE.** Dynamic single cell culture array. *Lab Chip,* 2006, vol. 6 (11), 1445-9
- **HAKIMI, N. et al.** Handheld skin printer: in situ formation of planar biomaterials and tissues. *Lab on a Chip,* 2018, vol. 18 (10), 1440-1451
- **KOBAYASHI, A. et al.** Preparation of stripe-patterned heterogeneous hydrogel sheets using microfluidic devices for high-density coculture of hepatocytes and fibroblasts. *Journal of Bioscience and Bioengineering,* 2013, vol. 116 (6), 761-767
- **TIAN, L. et al.** Spontaneous assembly of chemically encoded two-dimensional coacervate droplet arrays by acoustic wave patterning. *Nat Commun,* 2016, vol. 7, 13068
- **NICHOLS, M.K. et al.** Fabrication of Micropatterned Dipeptide Hydrogels by Acoustic Trapping of Stimulus-Responsive Coacervate Droplets. *Small,* 2018, vol. 14 (26), e1800739

- **OKOCHI, M. et al.** Three-dimensional cell culture array using magnetic force-based cell patterning for analysis of invasive capacity of BALB/3T3/v-src. *Lab Chip,* 2009, vol. 9 (23), 3378-84
- **OKOCHI, M. ; T. MATSUMURA ; H. HONDA.** Magnetic force-based cell patterning for evaluation of the effect of stromal fibroblasts on invasive capacity in 3D cultures. *Biosens Bioelectron,* 2013, vol. 42, 300-7
- **YAMAMOTO, S. et al.** Effect of vascular formed endothelial cell network on the invasive capacity of melanoma using the in vitro 3D co-culture patterning model. *PLoS One,* 2014, vol. 9 (7), e103502
- **LIN, R.Z. et al.** Magnetic reconstruction of three-dimensional tissues from multicellular spheroids. *Tissue Eng Part C Methods,* 2008, vol. 14 (3), 197-205
- **DEMRI, N. et al.** Remote Magnetic Microengineering and Alignment of Spheroids into 3D Cellular Fibers. *Advanced Functional Materials,* 2022, vol. 32 (50), 2204850
- **LEVARIO-DIAZ, V. et al.** Effect of acoustic standing waves on cellular viability and metabolic activity. *Sci Rep,* 2020, vol. 10 (1), 8493
- **NETO, A.I. et al.** Fabrication of Hydrogel Particles of Defined Shapes Using Superhydrophobic-Hydrophilic Micropatterns. *Adv Mater,* 2016, vol. 28 (35), 7613-9
- **JEON, N.L. et al.** Generation of solution and surface gradients using microfluidic systems. *Langmuir,* 2000, vol. 16 (22), 8311-8316
- **BEZENAH, J.R. ; Y.P. KONG ; A.J. PUTNAM.** Evaluating the potential of endothelial cells derived from human induced pluripotent stem cells to form microvascular networks in 3D cultures. *Scientific Reports,* 2018, vol. 8, 2671
- **GHAJAR, C.M. et al.** The effect of matrix density on the regulation of 3-D capillary morphogenesis. *Biophysical Journal,* 2008, vol. 94 (5), 1930-1941
- **NEWMAN, A.C. et al.** The requirement for fibroblasts in angiogenesis: fibroblast-derived matrix proteins are essential for endothelial cell lumen formation. *Molecular Biology of the Cell,* 2011, vol. 22 (20), 3791-3800